# EUROPEAN PATENT SPECIFICATION

(11) **EP 2 943 180 B1**
(45) Date of publication and mention of the grant of the patent: **04.09.2019**
(21) Application number: 14701433.6
(22) Date of filing: 09.01.2014
(51) Int. Cl.: A61K 8/368, A61K 8/73, A61Q 19/00, A61K 31/60, A61K 31/728

(54) **SOOTHING COSMETIC COMPOSITION BASED ON SALICYLIC ACID**
AUF SALICYLSÄURE AUFBAUENDE KOSMETISCHE ZUSAMMENSETZUNG ZUR BERUHIGUNG
COMPOSITION COSMÉTIQUE APAISANTE BASÉE SUR DE L'ACIDE SALICYLIQUE

(30) Priority: 10.01.2013 FR 1350214; 14.01.2013 US 201361751981 P
(43) Date of publication of application: 18.11.2015
(73) Proprietor: L'Oréal, 75008 Paris (FR)
(72) Inventor: PRUNEL, Anne, F-94100 Saint Maur (FR); DE POILLY, Valérie, F-94120 Fontenay Sous Bois (FR); PIERRE, Patricia, F-92160 Antony (FR)
(74) Representative: Nony
(86) International application number: PCT/IB2014/058147
(87) International publication number: WO 2014/108846

(56) References cited:
- EP-A1- 0 699 432
- EP-A2- 0 937 454
- FR-A1- 2 906 463
- FR-A1- 2 973 232
- JP-A- H11 180 832
- KR-A- 20030 062 285
- DATABASE GNPD [Online] MINTEL; July 2012 (2012-07), "24h T-Zone Care", Database accession no. 1818826

## Description

The present invention aims to provide a combination of active agents for preventing a modification of, and/or maintaining, skin homeostasis. It is directed in particular towards the prevention and/or treatment of skin irritations.

For the purposes of the invention, the term "skin" is intended to mean the whole of the skin of the human body, including the areas of the skin covered with body hair. Thus, in particular, the skin of the face, of the neckline, of the arms and of the legs, the lips and the scalp are targeted.

Human skin consists of three compartments, namely a superficial compartment, which is the epidermis, the dermis and a deep compartment, which is the hypodermis. The epidermis is a keratinized, stratified pavement epithelium. It consists mainly, i.e. 90%, of keratinocytes. As regards the dermis, it is a connective tissue which makes up the main bulk of the skin. It consists of collagen fibres and elastic fibres and also of glycosaminoglycans (GAGs) and of proteoglycans; its various structures form a complex network which plays a key role in the biomechanical properties of the skin.

The skin constitutes a barrier against external attacks. It is clear that the quality of the skin barrier and of the mucous membranes is affected daily following physiological or external stresses. Such stresses cause, at skin level, numerous modifications and degradations which result in irritation reactions, or even inflammatory reactions. Moreover, these inflammatory and/or irritation reactions may be accompanied by drying of the skin and are more widely involved in the overall modification of skin homeostasis.

Skin irritation is conventionally defined as a reversible and non-immunological local inflammatory reaction characterized by oedema and erythema induced after single or repeated contact of a chemical substance with the skin.

The biological events involved in skin irritation are complex and not very well described. It is known that keratinocytes play an important role in the initialization of the inflammatory skin reaction through the release of numerous mediators and of cytokines responsible for an entire inflammation cascade resulting in the clinical signs of irritation. Among the biological markers representative of an irritant or even inflammatory state, reference may more particularly be made to interleukin-1 (IL-1α) and to certain metabolic derivatives of arachidonic acid, such as the eicosanoids LTB4 and PGE2. The characterization of the level of expression of these compounds thus proves to be a reliable means for characterizing the development of an irritation reaction.

Physiologically, the signs of skin irritations can manifest themselves, depending on the strength of the irritation, through signs on the skin such as redness, dry patches, inflammatory erythema, oedema and/or spots. Irritated skin is skin which shows at least one of the following undesirable signs on the skin, namely redness, pruritus, dry patches, and/or which induces at least one feeling of discomfort, such as itching, tingling and tautness. Skin which is highly irritated can develop a skin reaction of inflammatory type, for instance inflammatory erythema, psoriasis, skin atopy, atopic dermatitis, an allergic reaction of immediate hypersensitivity type, such as urticaria, an allergic reaction of delayed hypersensitivity type, such as contact dermatitis, eczema, seborrhoeic dermatitis or acne.

Skin irritation is a very significant phenomenon. Greasy skin is in particular skin that is very sensitive and subject to redness and irritation.

The importance of being able to have products capable of preventing the manifestation of and/or treating undesirable skin disorders such as skin irritation reactions, in particular with an inflammatory nature, is thus understood.

There is therefore a need to have novel active agents capable of providing a feeling of comfort regarding skin which is in particular irritated.

There is also a need to have novel active agents capable of providing a soothing effect regarding skin which is in particular irritated.

For the purposes of the invention, the term "soothing" describes the ability of a compound to reduce the feeling of discomfort felt on the skin or the scalp, namely a feeling of itching, tingling and tautness, or to reduce redness of the skin.

There is also a need to have novel active agents capable of reducing the expression of IL-1α, LTB4 and PGE2.

An object of the present invention is to satisfy these needs.

More specifically, the present invention relates, according to one of its aspects, to a cosmetic and/or dermatological composition comprising, in a physiologically acceptable medium, at least 0.1% by weight of hyaluronic acid or a salt thereof, relative to the total weight of the composition, at least 0.1% by weight of salicylic acid relative to the total weight of the composition, and at least one lipophilic salicylic acid derivative of formula (I) 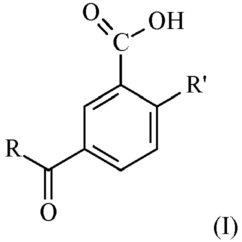 in which:
- the R radical denotes a linear, branched or cyclic, saturated aliphatic chain containing from 2 to 22 carbon atoms; an unsaturated chain containing from 2 to 22 carbon atoms and comprising one or more double bonds that may be conjugated; an aromatic ring bonded to the carbonyl radical directly or via saturated or unsaturated aliphatic chains containing from 2 to 7 carbon atoms; it being possible for said rings and chains to be substituted with one or more substituents, which may be identical or different, chosen from (a) halogen atoms, (b) a trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms;
- R' is a hydroxyl group or an ester group of formula: 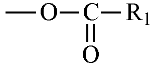 in which R₁ denotes a linear or branched, saturated or unsaturated aliphatic chain containing from 1 to 18 carbon atoms;
- and also salts thereof derived from an inorganic or organic base;
in which said hyaluronic acid or salt thereof (HA) and the salicylic acid (SA) are used in an (SA)/(HA) weight ratio of less than or equal to 3.

The term "lipophilic" salicylic acid derivative is intended to mean any salicylic acid derivative that is not soluble at 1% by weight in water and at 25°C.

Advantageously, the salicylic acid derivative is 5-n-octanoylsalicylic acid, also known as capryloyl salicylic acid.

Advantageously, this salicylic acid derivative is present in a proportion of at least 0.1% by weight relative to the total weight of the composition.

Advantageously, a composition according to the invention also comprises a secondary active agent for caring for greasy skin, chosen from desquamating agents and/or antimicrobial agents and/or anti-inflammatory agents and/or sebum regulators and/or antioxidants.

Against all expectations, the inventors have in fact noted that the combination of hyaluronic acid with salicylic acid and at least one salicylic acid derivative shows an advantageously synergistic soothing effect when these three compounds are used in amounts greater than those generally considered in the cosmetics industry for taking advantage of their respective property.

Indeed, hyaluronic acid is a compound that has already been proposed in cosmetic compositions, in particular as a moisturizing agent; as regards salicylic acid and the salicylic acid derivative, they are conventionally used as desquamating agents.

Salicylic acid and derivatives thereof have also been described in topical, in particular cosmetic, compositions, in particular for their bactericidal and/or keratolytic capacity.

The inventors thus recently observed that the combination of these compounds proves to be an active agent that is of use for modifying irritation processes or inflammatory processes. The combination according to the invention thus advantageously makes it possible to reduce, prevent and/or treat skin irritations, or skin disorders resulting from an inflammatory skin phenomenon.

In particular, the combination according to the invention proves to be quite particularly of use for maintaining the mechanism or mechanisms promoting the regulation of skin irritations. As emerges from the examples hereinafter, the inventors have shown that such a combination makes it possible to effectively reduce the expression of IL-1α, LTB4 and PGE2.

Thus, the present text describes the use of hyaluronic acid or a salt thereof in combination with salicylic acid and/or a salicylic acid derivative of formula (I) as described above, preferably 5-n-octanoylsalicylic acid, as a soothing active agent, in a cosmetic or dermatological composition.

It is also described the use of hyaluronic acid or a salt thereof in combination with salicylic acid and optionally at least one salicylic acid derivative of formula (I), and in particular 5-n-octanoylsalicylic acid, as a soothing active agent, in a cosmetic or dermatological composition, in particular dedicated to the treatment of greasy skin and/or acne-prone skin, and in particular for reducing skin discomfort and/or reducing and/or treating skin reactions in particular due to an irritating agent or to a factor of endogenous or exogenous origin.

The skin reactions are more particularly chosen from redness, itching and heating. For the purposes of the present invention, the term "redness" is intended to mean the redness which appears specifically at the place on the skin exhibiting an irritation. The redness targeted by the present invention does not relate to the diffuse redness associated with rosacea.

Thus, the present invention relates, according to another of its aspects, to a cosmetic, non therapeutic, use of hyaluronic acid or a salt thereof in combination with salicylic acid and/or a salicylic acid derivative of formula (I) as described above, preferably 5-n-octanoylsalicylic acid, as a soothing active agent.

According to another of is aspects, the present invention relates to hyaluronic acid or a salt thereof in combination with salicylic acid and/or a salicylic acid derivative of formula (I) as described above, preferably 5-n-octanoylsalicylic acid, as an active agent for use in a method for reducing irritation processes.

It is also described the use of hyaluronic acid or a salt thereof in combination with salicylic acid and optionally at least one salicylic acid derivative, preferably a lipophilic derivative and in particular 5-n-octanoylsalicylic acid, as an active agent for use for reducing irritation processes.

It is also described the non-therapeutic use of hyaluronic acid or a salt thereof in combination with salicylic acid and/or at least one salicylic acid derivative, preferably 5-n-octanoylsalicylic acid, as an active agent for use for improving the soothing effect of cosmetic and/or dermatological compositions dedicated to the treatment of greasy or acne-prone skin.

It is also described the non-therapeutic use of hyaluronic acid or a salt thereof in combination with salicylic acid and optionally at least one salicylic acid derivative, preferably a lipophilic salicylic acid derivative and in particular 5-n-octanoylsalicylic acid, as an active agent for use for improving the soothing effect of compositions dedicated to the treatment of greasy or acne-prone skin.

Preferably, the compounds are used in the amounts and/or the proportions by weight specified hereinafter.

The text also describes the use of hyaluronic acid or a salt thereof in combination with salicylic acid and optionally at least one salicylic acid derivative, preferably a lipophilic salicylic acid derivative and in particular 5-n-octanoylsalicylic acid, as an active agent that is of use for providing a feeling of comfort with regard to irritated skin.

For the purposes of the invention, irritated skin is skin which shows at least one of the undesirable skin signs such as tingling, pins and needles, itching, heating, feelings of discomfort, or feelings of tautness. In some cases, the signs of skin irritation may manifest themselves through redness, dry patches, erythema and/or spots. These signs are essentially dysaesthetic sensations.

It is also described the use of hyaluronic acid or a salt thereof in combination with salicylic acid and optionally at least one salicylic acid derivative, preferably a lipophilic salicylic acid derivative and in particular 5-n-octanoylsalicylic acid, as an active agent that is of use for combating phenomena of modification of homeostasis of the skin, in particular subjected to inflammatory processes.

It is also described the combination according to the invention as a dermatological active agent for preventing and/or treating an inflammatory condition of the skin.

For the purposes of the present invention, the term "preventing" is intended to mean reducing the risk of manifestation of the phenomenon under consideration. This reduction may be total or partial, i.e. result in a degree of risk that is lower than that pre-existing the use according to the invention.

For the purposes of the present invention, the term "treating" is intended to mean compensating for a physiological dysfunction and more generally reducing or even eliminating the undesirable disorder, the manifestation of which is in particular a consequence of this dysfunction.

For the purposes of the invention, the term "limiting" is intended to mean, from the viewpoint of a symptom, reducing the intensity of expression of this symptom, for example a skin irritation, and/or reducing the risk of occurrence of this symptom.

It it is understood that the reduction in the intensity and/or in the risk under consideration may be total or partial, i.e. the risk of occurrence of the symptom or the intensity of its expression remains, but to a lesser degree than that pre-existing the use according to the invention.

It is also described a cosmetic, or non-therapeutic, process for preventing the modification of, and/or maintaining, skin homeostasis in an individual in need thereof, comprising at least one step of administering to said subject an effective amount of the combination as defined above.

### COMBINATION ACCORDING TO THE INVENTION

In the descriptive part, the term "combination according to the invention" is intended to describe the simultaneous use of hyaluronic acid or a salt thereof (HA), of salicylic acid (SA) and/or optionally of at least one salicylic acid derivative of formula (I) (SAD). For the purposes of simplifying the reading, this combination can also be identified by (HA)/(SA)/(SAD).

### a) Hyaluronic acid or a salt thereof (HA)

Hyaluronic acid is a non-sulfated linear glycosaminoglycan composed of repeating D-glucuronic acid and N-acetyl-D-glucosamine units.

According to the invention, the hyaluronic acid derivative preferably has a number-average molecular weight ranging from 500 Da to 10 MDa and more particularly ranging from 2 KDa to 2 MDa.

As hyaluronic acid suitable for the present invention, mention may in particular be made of hyaluronic acids which are of animal origin, in their original form or crosslinked, such as those sold under the name Hylaform® by the company Genzyme, or else hyaluronic acids of genetic origin, including those intended for superficial wrinkles, wrinkles around the eyes or wrinkles around the mouth, such as those sold under the name Restylane Fine Lines® by Laboratoire Q-Med, or intended for deep wrinkles, and for lip and chin depressions and oval depressions of the face, such as those sold under the names Perlane® and Restylane Sub-Q® by Laboratoire Q-Med.

Preferably, as hyaluronic acid suitable for the present invention, mention may be made of those sold under the name Restylane® by Laboratoire Q-Med and under the name Surgiderm® by Laboratoire Corneal.

Mention may also be made of the low-molecular-weight (50 kDa) hyaluronic acid provided by Evonik under the name Hyacare 50®.

Among the hyaluronic acid salts, mention may in particular be made of the sodium salts, the potassium salts, the zinc salts and the silver salts, and mixtures thereof.

More particularly, as hyaluronic acid salts, mention may be made of potassium hyaluronate and sodium hyaluronate, preferably sodium hyaluronate.

The sodium hyaluronate of molecular weight 1 100 000 Daltons sold by the company Soliance under the name Cristalhyal® may also be considered in the context of the invention.

The present text describes the use of hyaluronic acid or a salt thereof at from 0.001% to 20% by weight, in particular from 0.01% to 15% by weight and more particularly from 0.1% to 10% by weight, relative to the total weight of a composition, and better still from 0.5% to 5%.

The cosmetic and/or dermatological composition as claimed according to the invention comprises at least 0.1% by weight of hyaluronic acid or a salt thereof, relative to the total weight of the composition, advantageously from 0.1% to 20% by weight, in particular from 0.1% to 15% by weight and more particularly from 0.1% to 10% by weight, relative to the total weight of the composition, and better still from 0.5% to 5%.

### b) Salicylic acid (SA) and derivatives thereof (SAD)

The lipophilic salicylic acid derivatives in accordance with the invention correspond to the formula (I) 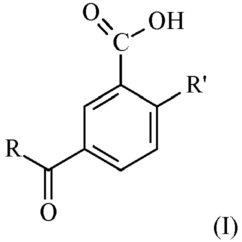 in which:
- the R radical denotes a linear, branched or cyclic, saturated aliphatic chain containing from 2 to 22 carbon atoms; an unsaturated chain containing from 2 to 22 carbon atoms and comprising one or more double bonds that may be conjugated; an aromatic ring bonded to the carbonyl radical directly or via saturated or unsaturated aliphatic chains containing from 2 to 7 carbon atoms; it being possible for said rings and chains to be substituted with one or more substituents, which may be identical or different, chosen from (a) halogen atoms, (b) a trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms;
- R' is a hydroxyl group or an ester group of formula: 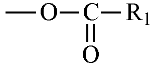 in which R₁ denotes a linear or branched, saturated or unsaturated aliphatic chain containing from 1 to 18 carbon atoms;
- and also salts thereof derived from an inorganic or organic base.

Preferentially, the R radical denotes a linear, branched or cyclic, saturated aliphatic chain containing from 3 to 11 carbon atoms; an unsaturated chain containing from 3 to 17 carbon atoms and comprising one or more conjugated or unconjugated double bonds; it being possible for said hydrocarbon-based chains to be substituted with one or more substituents, which may be identical or different, chosen from (a) halogen atoms, (b) a trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms;
- R' is a hydroxyl group or an ester group of formula: 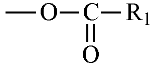 in which R₁ denotes a radical -(CH₂)ₙ-CH₃ where n is a number ranging from 0 to 14;
- and also salts thereof obtained by salification with an inorganic or organic base.

The compounds that are more particularly preferred are those in which the R radical is a C₃-C₁₁ alkyl group and R' denotes hydroxyl.

Other particularly advantageous compounds are those in which R represents a chain derived from linoleic, linolenic or oleic acid.

Another group of particularly preferred compounds is constituted of compounds in which the R radical denotes a C₃-C₁₁ alkyl group bearing a carboxyl function in free form or esterified with a lower alcohol containing from 1 to 6 carbon atoms and R' denotes hydroxyl. Lipophilic salicylic acid derivatives of formula (I) that may be used according to the invention are in particular described in patents US 6,159,479 and US 5,558,871, FR 2,581,542, US 4,767,750, EP 378 936, US 5,267,407, US 5,667,789, US 5,580,549 and EP-A-570,230.

Among the particularly preferred compounds of formula (I), mention may be made of 5-n-octanoylsalicylic acid (or capryloyl salicylic acid); 5-n-decanoylsalicylic acid; 5-n-dodecanoylsalicylic acid; 5-n-heptyloxysalicylic acid, and the corresponding salts thereof.

By way of salicylic acid derivatives, mention may be made of 5-n-octanoylsalicylic acid (or capryloyl salicylic acid), 5-n-decanoylsalicylic acid, 5-n-dodecanoylsalicylic acid and 5-n-heptyloxysalicylic acid.

The derivative in question is preferably 5-n-octanoylsalicylic acid.

For the purposes of the invention, the salts of these acids are also considered.

The salts can be obtained by salification of the acid under consideration with an organic or inorganic base. By way of inorganic base, mention may particularly be made of alkali metal or alkaline-earth metal hydroxylated bases, for instance sodium hydroxide or potassium hydroxide, and ammonia.

As regards the organic bases, they may in particular be bases of amine or alkanolamine type.

The present text describes the use of salicylic acid in a proportion of from 0.001% to 15% by weight of the total weight of the composition, in particular from 0.01% to 7% by weight and more preferentially from 0.1% to 5% by weight relative to the total weight of a composition, more particularly from 1% to 3%.

The cosmetic and/or dermatological composition as claimed according to the invention comprises at least 0.1% by weight of salicylic acid, relative to the total weight of the composition.

As regards the salicylic acid derivative, it may be present at from 0.001% to 10% by weight, in particular from 0.01% to 5% by weight and more preferentially from 0.1% to 1% by weight relative to the total weight of the composition.

According to one preferred embodiment variant, the hyaluronic acid or derivative (HA) and the salicylic acid (SA) are used in an (SA)/(HA) weight ratio at least equal to 1, or even greater than 1, and preferably greater than 1.2.

According to one preferred embodiment variant, the hyaluronic acid or derivative (HA) and the salicylic acid (SA) are used in an (SA)/(HA) weight ratio of less than or equal to 3 and preferably less than 2.

Thus, the cosmetic and/or dermatological composition as claimed in accordance with the invention comprises said hyaluronic acid or salt thereof (HA) and the salicylic acid (SA) in an (SA)(HA) weight ratio of less than or equal to 3 and preferably less than 2.

According to one preferred embodiment variant, the salicylic acid (SA) and the salicylic acid derivative (SAD) are used in an (SA)/(SAD) weight ratio ranging from 1.5 to 5 and preferably from 2 to 4.

According to one preferred embodiment variant, the hyaluronic acid or derivative (HA) and the salicylic acid derivative (SAD) are used in an (HA)/(SAD) weight ratio ranging from 0.5 to 5, preferably from 1 to 3 and more particularly from 1.5 to 2.5.

By way of illustration of combinations according to the invention that are particularly advantageous, mention may be made of those combining 0.5% to 2% by weight of hyaluronic acid or a salt thereof, 1% to 2% by weight of salicylic acid and 0.2% 1% by weight of a salicylic acid derivative, in particular 5-n-octanoylsalicylic acid.

### INDICATIONS

From a cosmetic point of view, the combination of the invention more particularly has the property of preventing and/or treating skin irritations.

Thus, generally, the combination of the invention can be used for preventing a modification of, and/or maintaining, skin homeostasis, in particular in the face of irritating phenomena and/or compounds.

According to one embodiment, the use of the invention can be more particularly dedicated to preventing a modification of, and/or reinforcing, the barrier function of skin chosen from weakened skin, aggravated skin, sensitive skin, irritable skin, reactive skin, intolerant skin, or greasy and/or acne-prone skin.

In other words, the combination considered according to the invention can advantageously make it possible to prevent the manifestation of, and/or reduce, the undesirable signs of skin irritation on weakened skin, aggravated skin, sensitive skin, irritable skin, reactive skin, intolerant skin, or greasy and/or acne-prone skin.

According to one embodiment, the use of the invention can be more particularly dedicated to providing a feeling of comfort regarding the signs of skin irritation and more particularly regarding weakened, aggravated, sensitive, irritable, reactive, intolerant or greasy and/or acne-prone skin.

Preferably, the combination of the invention can advantageously be used with regard to irritable and/or reactive, in particular greasy and/or acne-prone, skin.

More preferably, the invention can be used with regard to irritable and/or reactive and intolerant skin. Intolerant skin is skin that reacts, via sensations of heating, tautness, pins and needles and/or redness, to various factors such as the environment, emotions, foods and certain cosmetic products. These signs can generally be associated with hyperseborrhoeic or acneic skin with or without dry patches, and with erythema.

From a dermatological point of view, in the expression of an aggravated manifestation, a skin irritation may be reflected by the presence of symptoms of an inflammation. The combination of the invention can be used as an active agent in a dermatological or pharmaceutical composition.

Skin which is highly irritated can develop a skin reaction of inflammatory type, such as inflammatory erythema, psoriasis, skin atopy, atopic dermatitis, an allergic reaction of immediate hypersensitivity type, such as urticaria, an allergic reaction of delayed hypersensitivity type, for instance contact dermatitis, eczema, seborrhoeic dermatitis or acne.

The combination of the invention, from a dermatological or pharmaceutical point of view, can be used as an active agent for preventing and/or treating a skin condition chosen from erythema, atopic dermatitis, seborrhoeic dermatitis and acne.

### COMPOSITIONS

The (HA)/(SA)/(SAD) combination considered according to the invention may be advantageously formulated in a composition that may be in any galenical form normally available for the intended mode of administration.

A composition of the invention comprises a physiologically acceptable medium. It can be administered orally, parenterally, in particular subcutaneously or intradermally, or topically.

A composition of the invention can be a cosmetic, dermatological or pharmaceutical composition.

Preferably, a composition of the invention can be administered topically.

According to one embodiment, a composition of the invention administered topically may advantageously be formulated in any galenical form that is suitable for caring for the skin and mucous membranes and may be in the form of salves, creams, milks, ointments, powders, impregnated pads, solutions, gels, sprays, lotions or suspensions.

A topical composition may be either in anhydrous form or in aqueous form according to the dermocosmetic indication.

A composition intended for topical administration may be an aqueous, aqueous-alcoholic or oily solution, a solution or a dispersion of the lotion or serum type, an emulsion of liquid or semi-liquid consistency of the milk type, obtained by dispersing a fatty phase in an aqueous phase (O/W) or conversely (W/O), a suspension or an emulsion, of soft, semi-solid or solid consistency, of the cream type or of the aqueous or anhydrous gel type, a multiple emulsion (W/O/W or O/W/O), a microemulsion, a nanoemulsion, a preparation of microcapsules, a preparation of microparticles, a vesicular dispersion of ionic and/or non-ionic type, or a wax/aqueous phase dispersion.

According to one preferred embodiment, a topical composition may be in the form of a solution, a cream, a gel, an emulsion, a foam or an aerosol composition containing a propellant.

According to one preferred embodiment, a topical composition may also be in the form of a transdermal system allowing active or passive release of the active agent(s) transdermally, for example of the patch or patch gel (hydrogel) type.

These compositions are prepared according to the usual methods.

A composition according to the invention can constitute a composition for treating or caring for the skin or the scalp, or a sun protection or artificial tanning composition, or else a product for cleansing or removing makeup from the skin, a deodorant product or else a fragrancing compound.

Such a composition may then be uncoloured or weakly coloured, and may optionally contain additional cosmetic or dermatological active agents, in particular as indicated hereinafter. It may then be used as a care base for the skin or the lips, for example in the form of a lip balm, for protecting the lips against the cold and/or sunlight and/or the wind, such as a day or night care cream for facial and/or bodily skin.

It may also be in the form of a colouring or non-colouring, medicated or non-medicated shampoo, or a hair conditioner.

A composition according to the invention may also constitute a coloured cosmetic composition and in particular a makeup composition for the skin and/or mucous membranes, and in particular such a composition may be a foundation, optionally having care or treating properties.

A composition administered topically may in particular constitute a cleansing, protecting, treating or care cream, a skincare lotion, gel or foam, a cleansing or disinfecting lotion, a bath composition or a deodorant composition.

A composition according to the invention may also consist of a solid preparation constituting a soap or a cleansing bar.

When a composition of the invention is an emulsion, the proportion of the fatty phase may range from 5% to 80% by weight and preferably from 10% to 50% by weight relative to the total weight of the composition. The oils, emulsifiers and coemulsifiers used in the composition in emulsion form are chosen from those conventionally used in cosmetics and/or dermatology. The emulsifier and the coemulsifier may be present in the composition in a proportion ranging from 0.3% to 30% by weight and preferably from 0.5% to 20% by weight relative to the total weight of the composition.

When the composition of the invention is an oily solution or gel, the fatty phase may represent more than 90% of the total weight of the composition.

In a known manner, the galenical forms intended for topical administration may also contain adjuvants that are customary in the cosmetics, pharmaceutical and/or dermatology field, such as hydrophilic or lipophilic gelling agents, emulsifiers, hydrophilic or lipophilic active agents, preserving agents, antioxidants, solvents, fragrances, fillers, screening agents, odour absorbers and colorants. The amounts of these various adjuvants are those conventionally used in the field under consideration, for example from 0.01% to 20% of the total weight of the composition. Depending on their nature, these adjuvants may be introduced into the fatty phase and/or into the aqueous phase.

As fatty substances that may be used in the invention, mention may be made of mineral oils, for instance hydrogenated polyisobutene and liquid petroleum jelly, plant oils, for instance a liquid fraction of shea butter, sunflower oil and apricot kernel oil, animal oils, for instance perhydrosqualene, synthetic oils, in particular purcellin oil, isopropyl myristate and ethylhexyl palmitate, unsaturated fatty acids and fluoro oils, for instance perfluoropolyethers. It is also possible to use fatty alcohols, fatty acids, for instance stearic acid and, for example, waxes, in particular paraffin wax, carnauba wax and beeswax. It is also possible to use silicone compounds, for instance silicone oils, for example cyclomethicone and dimethicone, and silicone waxes, resins and gums.

As emulsifiers that can be used in the invention, mention may, for example, be made of esters of a fatty acid and of polyethylene glycol, esters of a fatty acid and of a polyol, such as glyceryl linoleate, glyceryl oleate, glyceryl linolenate, glyceryl stearate and sorbitan tristearate, polysorbate 60 and the PEG-6/PEG-32/Glycol Stearate mixture sold under the name Tefose 63 by the company Gattefosse.

As solvents that can be used in the invention, mention may be made of water, C₂-C₅ lower alcohols, in particular ethanol, isopropanol, and tert-butanol.

Preferably, a composition of the invention comprises a C₂-C₅ lower alcohol or a polyol.

A composition of the invention may also advantageously contain a spring and/or mineral water, chosen in particular from Vittel water, waters from the Vichy basin and La Roche Posay water.

Hydrophilic gelling agents that may be mentioned include carboxylic polymers such as carbomer, acrylic copolymers such as acrylate/alkyl acrylate copolymers, polyacrylamides and in particular the mixture of polyacrylamide, C₁₃₋₁₄ isoparaffin and laureth-7 sold under the name Sepigel 305® by the company SEPPIC, polysaccharides, for instance cellulose derivatives such as hydroxyalkylcelluloses and in particular hydroxypropylcellulose and hydroxyethylcellulose, natural gums such as guar gum, locust bean gum and xanthan gum, or clays.

Lipophilic gelling agents that may be mentioned include modified clays, for instance bentones, metal salts of fatty acids, for instance aluminium stearates and hydrophobic silica, or else ethylcellulose and polyethylene.

### Additional active agent

The combination according to the invention can be advantageously used with an additional active agent, in particular additional cosmetic, dermatological or pharmaceutical active agent.

Advantageously, such an additional cosmetic, dermatological or pharmaceutical active agent may be intended to exert a cosmetic, care or hygiene effect on the skin, the hair, the eyelashes, body hair and/or the scalp, and preferentially on the skin.

An additional active agent is advantageously chosen by those skilled in the art in such a way that it does not harm the effect of the active agent of the invention.

The choice of this active agent is of course conditioned by the effect jointly desired for the composition under consideration.

Thus, a composition according to the invention may also comprise an active agent for caring for greasy skin.

In the context of the present invention, the expression "active agent for caring for greasy skin" is intended to mean a compound which by itself, that is to say not requiring the intervention of an external agent to activate it, has an activity which may be in particular:
- a desquamating activity (in particular which allows the comedones to open), and/or
- an antimicrobial activity (in particular on P. acnes), and/or
- an anti-inflammatory activity, and/or
- a sebum-regulating activity, and/or
- an antioxidant activity (in particular which prevents squalene from being oxidized and comedones from being formed).

The active agent for caring for greasy skin may thus be chosen from desquamating agents and/or antimicrobial agents and/or anti-inflammatory agents and/or sebum regulators and/or antioxidants.

### Desquamating agents

The term "desquamating agent" is intended to mean any compound capable of acting:
- either directly on desquamation by promoting exfoliation, such as β-hydroxy acids; gentisic acid; oligofucoses; cinnamic acid; Saphora japonica extract; resveratrol and some jasmonic acid derivatives;
- or on the enzymes involved in desquamation or decomposition of the corneodesmosomes, glycosidases, stratum corneum chymotryptic enzyme (SCCE) or even other proteases (trypsin, chymotrypsin-like). Mention may be made of aminosulfonic compounds and in particular (N-2 hydroxyethylpiperazine-N-2-ethane)sulfonic acid (HEPES); derivatives of 2-oxothiazolidine-4-carboxylic acid (procysteine); derivatives of alpha-amino acids of glycine type (as described in EP-0 852 949, and also the sodium methyl glycine diacetate sold by BASF under the trade name Trilon M); honey; sugar derivatives, such as O-octanoyl-6-D-maltose and N-acetylglucosamine.

### Antimicrobial agents

The antimicrobial agents that may be used in the composition according to the invention are preferentially chosen from 2,4,4'-trichloro-2'-hydroxydiphenyl ether (or triclosan), 3,4,4'-trichlorocarbanilide, phenoxyethanol, phenoxypropanol, phenoxyisopropanol, hexamidine isethionate, metronidazole and salts thereof, miconazole and salts thereof, itraconazole, terconazole, econazole, ketoconazole, saperconazole, fluconazole, clotrimazole, butoconazole, oxiconazole, sulfaconazole, sulconazole, terbinafine, ciclopirox, ciclopirox olamine, undecylenic acid and salts thereof, benzoyl peroxide, 3-hydroxybenzoic acid, 4-hydroxybenzoic acid, phytic acid, N-acetyl-L-cysteine acid, lipoic acid, azelaic acid and salts thereof, arachidonic acid, resorcinol, 2,4,4'-trichloro-2'-hydroxydiphenyl ether, 3,4,4'-trichlorocarbanilide, octopirox, octoxyglycerine, octanoylglycine, caprylyl glycol, 10-hydroxy-2-decanoic acid, dichloro-phenylimidazoledioxolane and derivatives thereof described in patent WO 93/18743, copper pidolate, salicylic acid, zinc salicylate, iodopropynyl butylcarbamate, farnesol and phytosphingosines, and mixtures thereof.

### Anti-inflammatory agents

Advantageously, the anti-inflammatory or soothing agents that can be used in the composition according to the invention are chosen from pentacyclic triterpenes and plant extracts (e.g.: *Glycyrrhiza glabra*) containing same, for instance β-glycyrrhetinic acid and salts and/or derivatives thereof (glycyrrhetinic acid monoglucuronide, stearyl glycyrrhetinate, 3-stearoyloxyglycyrrhetic acid), ursolic acid and salts thereof, oleanolic acid and salts thereof, betulinic acid and salts thereof, bisabolol, an extract of *Paeonia suffruticosa* and/or *lactiflora,* salicylic acid salts and in particular zinc salicylate, phycosaccharides from the company Codif, an extract of *Laminaria saccharina*, canola oil, bisabolol and extracts of camomile, allantoin, Sepivital EPC (phosphoric diester of vitamins E and C) from SEPPIC, omega-3 unsaturated oils such as musk rose oil, blackcurrant oil, echium oil, fish oil, plankton extracts, capryloyl glycine, Seppicalm VG (sodium palmitoylproline and *Nymphaea alba*) from SEPPIC, an extract of *Pygeum,* an extract of *Boswellia serrata*, an extract of *Centipeda cunninghami*, an extract of *Helianthus annuus,* an extract of *Linum usitatissimum*, tocotrienols, extracts of *Cola nitida*, extracts of *Centella asiatica,* piperonal, an extract of clove, an extract of *Epilobium angustifolium*, aloe vera, an extract of *Bacopa monieri*, phytosterols, cortisone, hydrocortisone, indomethacin and betamethasone.

### Sebum regulators

When the composition according to the invention comprises a sebum regulator such as a 5α-reductase inhibitor, this agent is advantageously chosen from:
- retinoids, and in particular retinol;
- sulfur and sulfur derivatives;
- zinc salts such as zinc lactate, gluconate, pidolate, carboxylate, salicylate and/or cysteate;
- selenium chloride;
- vitamin B6 or pyridoxine;
- the mixture of capryloyl glycine, sarcosine and extract of *Cinnamomum zeylanicum* sold in particular by the company SEPPIC under the trade name Sepicontrol A5®;
- an extract of *Laminaria saccharina* sold in particular by the company SECMA under the trade name Phlorogine®;
- an extract of *Spiraea ulmaria* sold in particular by the company Silab under the trade name Sebonormine®;
- extracts of plants of the species *Arnica montana, Cinchona succirubra, Eugenia caryophyllata, Humulus lupulus, Hypericum perforatum, Mentha piperita, Rosmarinus officinalis, Salvia oficinalis* and *Thymus vulgaris,* all sold, for example, by the company Maruzen;
- an extract of *Serenoa repens* sold in particular by the company Euromed;
- extracts of plants of the genus *Silybum*;
- plant extracts containing sapogenins and in particular extracts of *Dioscorea* plants rich in diosgenin; and
- extracts of *Eugenia caryophyllata* containing eugenol and eugenyl glucoside.

### Antioxidants

The antioxidants that are preferred for use in the present invention are advantageously chosen from tocopherol and esters thereof, such as tocopheryl acetate; BHT and BHA.

According to one advantageous embodiment, a composition according to the invention contains at least one other active agent chosen from anti-UV screening agents, moisturizers, depigmenting agents, anti-glycation agents, NO-synthase inhibitors, agents for stimulating the synthesis of dermal or epidermal macromolecules and/or for preventing their degradation, agents for stimulating fibroblast or keratinocyte proliferation, muscle relaxants or dermo-decontracting agents, tensioning agents, agents for combating pollution, free-radical scavengers, soothing agents, desquamating agents and active agents that act on the energy metabolism of cells.

Needless to say, a person skilled in the art will take care to select the optional active agent(s) added to the composition according to the invention in such a way that the advantageous properties intrinsically associated with the composition in accordance with the invention are not, or are not substantially, adversely affected by the envisaged addition.

These active agents are generally present in the composition in a content ranging from 0.0001% to 20% by weight and preferably from 0.01% to 10% by weight relative to the total weight of said composition.

The compositions according to the invention may be manufactured via the known processes, generally used in the cosmetics or dermatology field.

The examples hereinafter are given as non-limiting illustrations of the field of the invention.

The percentages are expressed therein by weight of starting materials.

The compounds are, as appropriate, mentioned as chemical names or as CTFA (International Cosmetic Ingredient Dictionary and Handbook) names.

### EXAMPLE 1

**Composition according to the invention of gel type**

| CHEMICAL NAME | CONCENTRATION |
|---|---|
| 1,2-OCTANEDIOL | 0.3 |
| POLY ACRYLAMIDOMETHYLPROPANESULFONIC ACID | 2 |
| PARTIALLY NEUTRALIZED | |
| WITH AQUEOUS AMMONIA | |
| AND CROSSLINKED | |
| HOSTACERIN AMPS® from CLARIANT | |
| 5-n-OCTANOYLSALICYLIC ACID | 0.5 |
| SALICYLIC ACID POWDER | 1.5 |
| NOVACYL | |
| 2-OCTYLDODECANOL | 0.5 |
| TRIETHANOLAMINE | 1.84 |
| FRAGRANCE | 0.1 |
| SODIUM HYALURONATE | 1 |
| (MW: 1 100 000) POWDER | |
| CRISTALHYAL® from SOLIANCE | |
| ETHYLENEDIAMINETETRAACETIC ACID DISODIUM SALT, 2 H₂O | 0.2 |
| GLYCEROL | 5 |
| ETHYL ALCOHOL | 8 |
| WATER | qs 100 |

The formula is prepared using a deflocculating device (mixer), the starting materials are added successively, so as to obtain a thick translucent gel.

### Evaluation method:

Monitoring of the change in treated and untreated skin by an expert and self-evaluation questionnaire.

The study is carried out on a panel of 36 women (18-45 years old) with greasy-to-mixed and acne-prone skin. The treatment consists of an application twice a day (morning and evening) for 8 days, followed by 7 days without treatment.

The composition obtained has a significant effectiveness on redness as early as day 2, and on skin imperfections as early as day 3.

The self-evaluation questionnaire confirms these excellent results and demonstrates the soothing effectiveness of the product.

### EXAMPLE 2

**Composition according to the invention of gel type**

| | |
|---|---|
| CAPRYLOYL SALICYLIC ACID | 0.5 |
| TRIETHANOLAMINE | 1.8 |
| SALICYLIC ACID | 1.5 |
| SODIUM HYALURONATE (CRISTALHYAL® from SOLIANCE) | 1 |
| AMMONIUM POLY ACRYLDIMETHYL TAURAMIDE | 2 |
| HOSTACERIN AMPS from CLARIANT | |
| ETHANOL | 5 |
| WATER | qs 100 |

### EXAMPLE 3

**Composition of O/W emulsion type**

| | |
|---|---|
| TERASODIUM EDTA | 0.2 |
| CAPRYLOYL SALICYLIC ACID | 1 |
| TRIETHANOLAMINE | 0.6 |
| KAOLIN | 1 |
| SILICA | 1 |
| SALICYLIC ACID | 1.5 |
| PENT AERYTHRITYL TETRAETHYLHEXONOATE | 3 |
| PARAFFIN | 1 |
| CETYL ALCOHOL | 1.6 |
| ISOPROPYL LAUROYL SARCOSINATE | 3 |
| SODIUM HYALURONATE (CRISTALHYAL® from SOLIANCE) | 2 |
| ACRYLATES/C10-30 ALKYL ACRYLATE CROSSPOLYMER | 0.3 |
| AMMONIUM POLYACRYLDIMETHYL TAURAMIDE (HOSTACERIN AMPS from CLARIANT) | 0.3 |
| NYLON-12 | 1.5 |
| SODIUM POLYACRYLATE | 0.5 |
| DIMETHICONE | 4 |
| DIMETHICONE (and) DIMETICONE/VINYL DIMETHICONE CROSSPOLYMER | 3 |
| DIMETHICONE (and) DIMETHICONOL | 1 |
| ETHANOL | 5 |
| GLYCEROL | 5 |
| WATER qs | 100 |

### EXAMPLE 4

**Composition of cleansing gel type**

| | |
|---|---|
| DISODIUM EDTA | 0.1 |
| SODIUM LACTATE | 1 |
| CAPRYLOYL SALICYLIC ACID | 0.8 |
| TRIETHANOLAMINE | 0.4 |
| SALICYLIC ACID | 1.2 |
| ISOPROPYL LAUROYL SARCOSINATE | 0.5 |
| SODIUM HYALURONATE | 0.8 |
| SODIUM POLYACRYLATE | 0.5 |
| AMMONIUM ACRYLOYLDIMETHYLTAURATE/STEARETH - 25 METHACRYLATE CROSSPOLYMER | 1.3 |
| ETHANOL | 2 |
| GLYCEROL | 0.5 |
| DISODIUM COCOAMPHODIACETATE | 0.42 |
| POLOXAMER 188 | 1 |
| WATER | qs 100 |

### EXAMPLE 5

### Demonstration of the soothing effectiveness of the combination of the invention

The test consists in evaluating the effect of the combination on the inhibition of the response of superficial epidermal cells which are in an inflammatory state, by assaying IL-1α, LTB4 or PGE2 released by normal human epidermal keratinocytes (NHEKs) after stimulation with the agent capable of causing a skin irritation.

The cells are a keratinocyte line of human origin (HL60). They are cultured in RPMI 1640 medium (Life Technologies) supplemented with 2mM of L-glutamine, 50 U/ml of penicillin, 50 µg/ml of streptomycin and 10% of foetal calf serum (FCS). The culturing is carried out at 37°C and 5% CO₂.

In the case of IL-1α, the cells are preincubated for 24 hours in the presence of phorbol myristate acetate and of calcium ionophore A23187 (Sigma reference C752).

In the case of LTB4, the cells are preincubated for 24 hours in the presence of arachidonic acid and of calcium ionophore A23187 (Sigma reference C752).

In the case of PEG2, the cells are preincubated for 24 hours in the presence of phorbol myristate acetate.

The media are then replaced with the same media but containing the compounds tested (cf. results table).

At the end of this new incubation period (15 min, 37°C), the supernatants are collected and an ELISA assay (R and D Systems Kit, reference DE0275) of the amount of IL-1α, LTB4 or PGE2 released into the culture medium is carried out.

For each test, a control without the tested compounds is carried out. The table indicates the decrease or increase in the production of IL-1α, LTB4 or PGE2 relative to the control.

| | IL-1α | LTB4 | PGE2 |
|---|---|---|---|
| 0.002% Salicylic acid | 11% | 18% | -7% |
| 0.0002% Salicylic acid | -2% | 14% | 15% |
| 0.0022% Hyaluronic acid | -4% | -12% | -12% |
| 0.00074% Hyaluronic acid | -5% | 15% | -22% |
| 0.002% Salicylic acid + 0.0022% Hyaluronic acid | -8% | -14% | -35% |
| 0.0002% Salicylic acid + 0.00074% Hyaluronic acid | -14% | -16% | -50% |

Under the experimental conditions retained, the compositions containing the combination of salicylic acid and hyaluronic acid result in a very clear decrease in the production of IL-1α, LTB4 or PGE2, reflecting a soothing effect. Salicylic acid combined with hyaluronic acid makes it possible to potentiate these effects on the Il-1α, LTB4 and PGE2 markers compared with each one of the compounds.

## Claims

1. Cosmetic and/or dermatological composition comprising, in a physiologically acceptable medium, at least 0.1% by weight of hyaluronic acid or a salt thereof, relative to the total weight of the composition, at least 0.1% by weight of salicylic acid relative to the total weight of the composition, and at least one lipophilic salicylic acid derivative of formula (I) 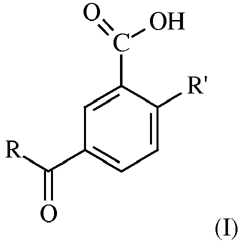 in which:
- the R radical denotes a linear, branched or cyclic, saturated aliphatic chain containing from 2 to 22 carbon atoms; an unsaturated chain containing from 2 to 22 carbon atoms and comprising one or more double bonds that may be conjugated; an aromatic ring bonded to the carbonyl radical directly or via saturated or unsaturated aliphatic chains containing from 2 to 7 carbon atoms; it being possible for said rings and chains to be substituted with one or more substituents, which may be identical or different, chosen from (a) halogen atoms, (b) a trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms;
- R' is a hydroxyl group or an ester group of formula: 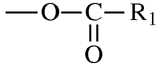 in which R₁ denotes a linear or branched, saturated or unsaturated aliphatic chain containing from 1 to 18 carbon atoms;
- and also salts thereof derived from an inorganic or organic base;
in which said hyaluronic acid or salt thereof (HA) and the salicylic acid (SA) are used in an (SA)/(HA) weight ratio of less than or equal to 3.

2. Composition according to Claim 1, in which said salicylic acid derivative is of formula (I) in which:
- the R radical denotes a linear, branched or cyclic, saturated aliphatic chain containing from 3 to 11 carbon atoms; an unsaturated chain containing from 3 to 17 carbon atoms and comprising one or more conjugated or unconjugated double bonds; it being possible for said hydrocarbon-based chains to be substituted with one or more substituents, which may be identical or different, chosen from (a) halogen atoms, (b) a trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms;
- R' is a hydroxyl group or an ester group of formula: 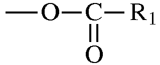 in which R₁ denotes a radical -(CH₂)ₙ-CH₃ where n is a number ranging from 0 to 14;
- and also salts thereof obtained by salification with an inorganic or organic base.

3. Composition according to Claim 1, in which said salicylic acid derivative is 5-n-octanoylsalicylic acid.

4. Composition according to any one of the preceding claims, in which said salicylic acid derivative is present in a proportion of at least 0.1% by weight relative to the total weight of said composition.

5. Composition according to any one of the preceding claims, in which said hyaluronic acid is present in a proportion of from 0.1% to 10% by weight relative to the total weight of the composition.

6. Composition according to any one of the preceding claims, in which said salicylic acid is present in a proportion of from 0.1% to 5% by weight relative to the total weight of the composition.

7. Composition according to any one of the preceding claims, in which said salicylic acid derivative is present in a proportion of from 0.1% to 1% by weight relative to the total weight of the composition.

8. Composition according to any one of the preceding claims, in which said hyaluronic acid or salt thereof (HA) and said salicylic acid (SA) are used in an (SA)/(HA) weight ratio of less than 2.

9. Composition according to any one of the preceding claims, also comprising a C₂-C₅ lower alcohol or a polyol.

10. A cosmetic, non therapeutic, use of hyaluronic acid or a salt thereof in combination with salicylic acid and/or a salicylic acid derivative, preferably 5-n-octanoylsalicylic acid, as a soothing active agent;
wherein the salicylic acid derivative is of formula (I) 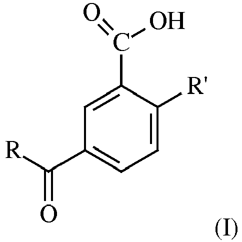 in which:
- the R radical denotes a linear, branched or cyclic, saturated aliphatic chain containing from 2 to 22 carbon atoms; an unsaturated chain containing from 2 to 22 carbon atoms and comprising one or more double bonds that may be conjugated; an aromatic ring bonded to the carbonyl radical directly or via saturated or unsaturated aliphatic chains containing from 2 to 7 carbon atoms; it being possible for said rings and chains to be substituted with one or more substituents, which may be identical or different, chosen from (a) halogen atoms, (b) a trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms;
- R' is a hydroxyl group or an ester group of formula: 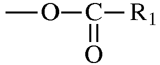 in which R₁ denotes a linear or branched, saturated or unsaturated aliphatic chain containing from 1 to 18 carbon atoms;
- and also salts thereof derived from an inorganic or organic base.

11. A hyaluronic acid or a salt thereof in combination with salicylic acid and/or a salicylic acid derivative, preferably 5-n-octanoylsalicylic acid, as an active agent for use in a method for reducing irritation processes;
wherein the salicylic acid derivative is of formula (I) 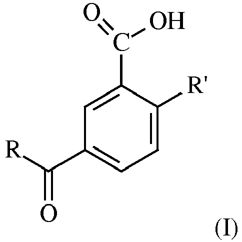 in which:
- the R radical denotes a linear, branched or cyclic, saturated aliphatic chain containing from 2 to 22 carbon atoms; an unsaturated chain containing from 2 to 22 carbon atoms and comprising one or more double bonds that may be conjugated; an aromatic ring bonded to the carbonyl radical directly or via saturated or unsaturated aliphatic chains containing from 2 to 7 carbon atoms; it being possible for said rings and chains to be substituted with one or more substituents, which may be identical or different, chosen from (a) halogen atoms, (b) a trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms;
- R' is a hydroxyl group or an ester group of formula: 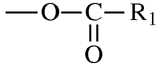 in which R₁ denotes a linear or branched, saturated or unsaturated aliphatic chain containing from 1 to 18 carbon atoms;
- and also salts thereof derived from an inorganic or organic base.

## Patentansprüche

1. Kosmetische und/oder dermatologische Zusammensetzung, umfassend, in einem physiologisch annehmbaren Medium, mindestens 0,1 Gew.-% Hyaluronsäure oder ein Salz davon relativ zu dem Gesamtgewicht der Zusammensetzung, mindestens 0,1 Gew.-% Salicylsäure relativ zu dem Gesamtgewicht der Zusammensetzung und mindestens ein lipophiles Salicylsäurederivat aus Formel (I) 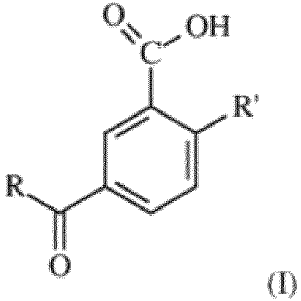 in der:
- das R-Radikal eine lineare, verzweigte oder cyclische gesättigte aliphatische Kette bezeichnet, die von 2 bis 22 Kohlenstoffatome enthält; wobei eine ungesättigte Kette von 2 bis 22 Kohlenstoffatome enthält und eine oder mehrere Doppelbindungen umfasst, die konjugiert sein können; wobei ein aromatischer Ring an das Carbonylradikal direkt oder über gesättigte oder ungesättigte aliphatische Ketten gebunden ist, die von 2 bis 7 Kohlenstoffatome enthalten; wobei es möglich ist, dass die Ringe und Ketten mit einem oder mehreren Substituenten substituiert sind, die identisch oder unterschiedlich sein können, ausgewählt aus (a) Halogenatomen, (b) einer Trifluormethylgruppe, (c) Hydroxylgruppen in freier Form oder verestert mit einer Säure, die von 1 bis 6 Kohlenstoffatome enthält, oder (d) einer Carboxylfunktion in freier Form oder verestert mit einem niederen Alkohol, der von 1 bis 6 Kohlenstoffatome enthält;
- R' eine Hydroxylgruppe oder eine Estergruppe aus folgender Formel ist: 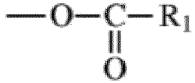 in der R₁ eine lineare oder verzweigte, gesättigte oder ungesättigte aliphatische Kette bezeichnet, die von 1 bis 18 Kohlenstoffatome enthält;
- und außerdem Salze davon, die von einer anorganischen oder organischen Base abgeleitet sind;
in der die Hyaluronsäure oder das Salz davon (HA) und die Salicylsäure (SA) in einem (SA)/(HA)-Gewichtsverhältnis von weniger als oder gleich 3 verwendet werden.

2. Zusammensetzung nach Anspruch 1, in der das Salicylsäurederivat aus Formel (I) ist, in der:
- das R-Radikal eine lineare, verzweigte oder cyclische gesättigte aliphatische Kette bezeichnet, die von 3 bis 11 Kohlenstoffatome enthält; wobei eine ungesättigte Kette von 3 bis 17 Kohlenstoffatome enthält und eine oder mehrere konjugierte oder dekonjugierte Doppelbindungen umfasst; wobei es möglich ist, dass die Ketten auf Kohlenwasserstoffbasis mit einem oder mehreren Substituenten substituiert sind, die identisch oder unterschiedlich sein können, ausgewählt aus (a) Halogenatomen, (b) einer Trifluormethylgruppe, (c) Hydroxylgruppen in freier Form oder verestert mit einer Säure, die von 1 bis 6 Kohlenstoffatome enthält, oder (d) einer Carboxylfunktion in freier Form oder verestert mit einem niederen Alkohol, der von 1 bis 6 Kohlenstoffatome enthält;
- R' eine Hydroxylgruppe oder eine Estergruppe aus folgender Formel ist: 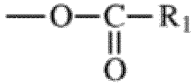 in der R₁ ein -(CH₂)ₙ-CH₃-Radikal bezeichnet, wobei n eine Zahl im Bereich von 0 bis 14 ist;
- und außerdem Salze davon, die durch Salzbildung mit einer anorganischen oder organischen Base erhalten werden.

3. Zusammensetzung nach Anspruch 1, in der das Salicylsäurederivat 5-n-Octanoylsalicylsäure ist.

4. Zusammensetzung nach einem der vorhergehenden Ansprüche, in der das Salicylsäurederivat in einem Anteil von mindestens 0,1 Gew.-% relativ zu dem Gesamtgewicht der Zusammensetzung vorhanden ist.

5. Zusammensetzung nach einem der vorhergehenden Ansprüche, in der die Hyaluronsäure in einem Anteil von 0,1 Gew.-% bis 10 Gew.-% relativ zu dem Gesamtgewicht der Zusammensetzung vorhanden ist.

6. Zusammensetzung nach einem der vorhergehenden Ansprüche, in der die Salicylsäure in einem Anteil von 0,1 Gew.-% bis 5 Gew.-% relativ zu dem Gesamtgewicht der Zusammensetzung vorhanden ist.

7. Zusammensetzung nach einem der vorhergehenden Ansprüche, in der das Salicylsäurederivat in einem Anteil von 0,1 Gew.-% bis 1 Gew.-% relativ zu dem Gesamtgewicht der Zusammensetzung vorhanden ist.

8. Zusammensetzung nach einem der vorhergehenden Ansprüche, in der die Hyaluronsäure oder das Salz davon (HA) und die Salicylsäure (SA) in einem (SA)/(HA)-Gewichtsverhältnis von weniger als 2 verwendet werden.

9. Zusammensetzung nach einem der vorhergehenden Ansprüche, die außerdem einen niederen C₂-C₅-Alkohol oder ein Polyol umfasst.

10. Kosmetische, nicht-therapeutische Verwendung von Hyaluronsäure oder einem Salz davon in Kombination mit Salicylsäure und/oder einem Salicylsäurederivat, bevorzugt 5-n-Octanoylsalicylsäure, als einen beruhigenden Wirkstoff;
wobei das Salicylsäurederivat aus Formel (I) ist 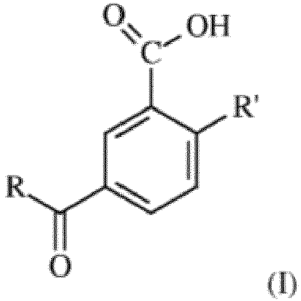 in der:
- das R-Radikal eine lineare, verzweigte oder cyclische gesättigte aliphatische Kette bezeichnet, die von 2 bis 22 Kohlenstoffatome enthält; wobei eine ungesättigte Kette von 2 bis 22 Kohlenstoffatome enthält und eine oder mehrere Doppelbindungen umfasst, die konjugiert sein können; wobei ein aromatischer Ring an das Carbonylradikal direkt oder über gesättigte oder ungesättigte aliphatische Ketten gebunden ist, die von 2 bis 7 Kohlenstoffatome enthalten; wobei es möglich ist, dass die Ringe und Ketten mit einem oder mehreren Substituenten substituiert sind, die identisch oder unterschiedlich sein können, ausgewählt aus (a) Halogenatomen, (b) einer Trifluormethylgruppe, (c) Hydroxylgruppen in freier Form oder verestert mit einer Säure, die von 1 bis 6 Kohlenstoffatome enthält, oder (d) einer Carboxylfunktion in freier Form oder verestert mit einem niederen Alkohol, der von 1 bis 6 Kohlenstoffatome enthält;
- R' eine Hydroxylgruppe oder eine Estergruppe aus folgender Formel ist: 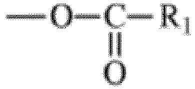 in der R₁ eine lineare oder verzweigte, gesättigte oder ungesättigte aliphatische Kette bezeichnet, die von 1 bis 18 Kohlenstoffatome enthält;
- und außerdem Salze davon, die von einer anorganischen oder organischen Base abgeleitet sind.

11. Hyaluronsäure oder ein Salz davon in Kombination mit Salicylsäure und/oder einem Salicylsäurederivat, bevorzugt 5-n-Octanoylsalicylsäure, als ein Wirkstoff zur Verwendung in einem Verfahren zum Verringern von Reizungsvorgängen;
wobei das Salicylsäurederivat aus Formel (I) ist 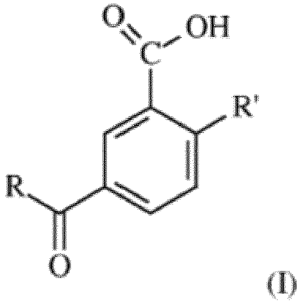 in der:
- das R-Radikal eine lineare, verzweigte oder cyclische gesättigte aliphatische Kette bezeichnet, die von 2 bis 22 Kohlenstoffatome enthält; wobei eine ungesättigte Kette von 2 bis 22 Kohlenstoffatome enthält und eine oder mehrere Doppelbindungen umfasst, die konjugiert sein können; wobei ein aromatischer Ring an das Carbonylradikal direkt oder über gesättigte oder ungesättigte aliphatische Ketten gebunden ist, die von 2 bis 7 Kohlenstoffatome enthalten; wobei es möglich ist, dass die Ringe und Ketten mit einem oder mehreren Substituenten substituiert sind, die identisch oder unterschiedlich sein können, ausgewählt aus (a) Halogenatomen, (b) einer Trifluormethylgruppe, (c) Hydroxylgruppen in freier Form oder verestert mit einer Säure, die von 1 bis 6 Kohlenstoffatome enthält, oder (d) einer Carboxylfunktion in freier Form oder verestert mit einem niederen Alkohol, der von 1 bis 6 Kohlenstoffatome enthält;
- R' eine Hydroxylgruppe oder eine Estergruppe aus folgender Formel ist: 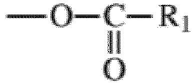 in der R₁ eine lineare oder verzweigte, gesättigte oder ungesättigte aliphatische Kette bezeichnet, die von 1 bis 18 Kohlenstoffatome enthält;
- und außerdem Salze davon, die von einer anorganischen oder organischen Base abgeleitet sind.

## Revendications

1. Composition cosmétique et/ou dermatologique comprenant, dans un milieu physiologiquement acceptable, au moins 0,1 % en poids d'acide hyaluronique ou d'un de ses sels par rapport au poids total de la composition, au moins 0,1 % en poids d'acide salicylique par rapport au poids total de la composition, et au moins un dérivé lipophile d'acide salicylique de formule (I) 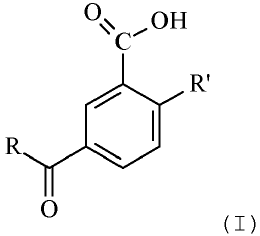 dans laquelle :
- le radical R désigne une chaîne aliphatique ayant de 2 à 22 atomes de carbone, saturée, linéaire, ramifiée ou cyclique ; une chaîne insaturée ayant de 2 à 22 atomes de carbone contenant une ou plusieurs doubles liaisons pouvant être conjuguées ; un noyau aromatique lié au radical carbonyle directement ou par l'intermédiaire de chaînes aliphatiques saturées ou insaturées ayant de 2 à 7 atomes de carbone ; lesdits noyaux et chaînes pouvant être substitués par un ou plusieurs substituants, identiques ou différents, choisis parmi (a) les atomes d'halogène, (b) le groupe trifluorométhyle, (c) des groupes hydroxyle sous forme libre ou estérifiée par un acide ayant de 1 à 6 atomes de carbone, ou (d) une fonction carboxyle sous forme libre ou estérifiée par un alcool inférieur ayant de 1 à 6 atomes de carbone ;
- R' est un groupe hydroxyle ou un groupe ester de formule 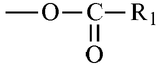 dans laquelle R₁ désigne une chaîne aliphatique, saturée ou insaturée, linéaire ou ramifiée contenant de 1 à 18 atomes de carbone ;
- ainsi que ses sels issus d'une base minérale ou organique ;
et dans laquelle ledit acide hyaluronique ou sel de celui-ci (HA) et l'acide salicylique (SA) sont utilisés en un rapport en poids (SA)/(HA) inférieur ou égal à 3.

2. Composition selon la revendication 1, dans laquelle ledit dérivé d'acide salicylique est de formule (I) dans laquelle :
- le radical R désigne une chaîne aliphatique saturée, linéaire, ramifiée ou cyclique contenant de 3 à 11 atomes de carbone ; une chaîne insaturée contenant de 3 à 17 atomes de carbone et comprenant une ou plusieurs doubles liaisons conjuguées ou non ; lesdites chaînes hydrocarbonées pouvant être substituées par un ou plusieurs substituants, identiques ou différents, choisis parmi (a) les atomes d'halogène (b) le groupe trifluorométhyle, (c) des groupes hydroxyle sous forme libre ou estérifiée par un acide ayant de 1 à 6 atomes de carbone ou (d) une fonction carboxyle sous forme libre ou estérifiée par un alcool inférieur ayant de 1 à 6 atomes de carbone ;
- R' est un groupe hydroxyle ou groupe ester de formule : 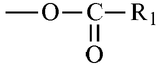 dans laquelle R₁ désigne un radical -(CH₂)ₙ-CH₃ où n est un nombre allant de 0 à 14 ;
- ainsi que leurs sels obtenus par salification par une base minérale ou organique.

3. Composition selon la revendication 1, dans laquelle ledit dérivé d'acide salicylique est l'acide 5-n-octanoylsalicylique.

4. Composition selon l'une quelconque des revendications précédentes, dans laquelle ledit dérivé d'acide salicylique est présent à raison d'au moins 0,1 % en poids par rapport au poids total de ladite composition.

5. Composition selon l'une quelconque des revendications précédentes, dans laquelle ledit acide hyaluronique est présent à raison de 0,1 à 10 % en poids par rapport au poids total de la composition.

6. Composition selon l'une quelconque des revendications précédentes, dans laquelle ledit acide salicylique est présent à raison de 0,1 % à 5 % en poids par rapport au poids total de la composition.

7. Composition selon l'une quelconque des revendications précédentes, dans laquelle ledit dérivé d'acide salicylique est présent à raison de 0,1 % à 1 % en poids par rapport au poids total de la composition.

8. Composition selon l'une quelconque des revendications précédentes, dans laquelle ledit acide hyaluronique ou sel de celui-ci (HA) et ledit acide salicylique (SA) sont utilisés en un rapport en poids (SA)/(HA) inférieur à 2.

9. Composition selon l'une quelconque des revendications précédentes, comprenant en outre un alcool inférieur en C₂-C₅ ou un polyol.

10. Utilisation cosmétique non thérapeutique d'acide hyaluronique ou d'un de ses sels en combinaison avec de l'acide salicylique et/ou un dérivé d'acide salicylique, de préférence l'acide 5-n-octanoylsalicylique, en tant que principe actif apaisant ; dans laquelle le dérivé d'acide salicylique est de formule (I) 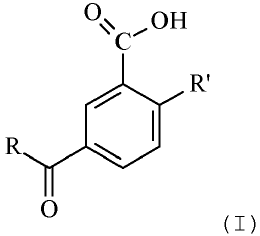 dans laquelle :
- le radical R désigne une chaîne aliphatique ayant de 2 à 22 atomes de carbone, saturée, linéaire, ramifiée ou cyclique ; une chaîne insaturée ayant de 2 à 22 atomes de carbone contenant une ou plusieurs doubles liaisons pouvant être conjuguées ; un noyau aromatique lié au radical carbonyle directement ou par l'intermédiaire de chaînes aliphatiques saturées ou insaturées ayant de 2 à 7 atomes de carbone ; lesdits noyaux et chaînes pouvant être substitués par un ou plusieurs substituants, identiques ou différents, choisis parmi (a) les atomes d'halogène, (b) le groupe trifluorométhyle, (c) des groupes hydroxyle sous forme libre ou estérifiée par un acide ayant de 1 à 6 atomes de carbone, ou (d) une fonction carboxyle sous forme libre ou estérifiée par un alcool inférieur ayant de 1 à 6 atomes de carbone ;
- R' est un groupe hydroxyle ou un groupe ester de formule 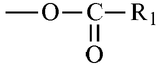 dans laquelle R₁ désigne une chaîne aliphatique, saturée ou insaturée, linéaire ou ramifiée contenant de 1 à 18 atomes de carbone ;
- ainsi que ses sels issus d'une base minérale ou organique.

11. Acide hyaluronique ou un de ses sels en combinaison avec de l'acide salicylique et/ou un dérivé d'acide salicylique, de préférence l'acide 5-n-octanoylsalicylique, en tant que principe actif pour une utilisation dans un procédé pour réduire les processus d'irritation ;
dans lequel le dérivé d'acide salicylique est de formule (I) 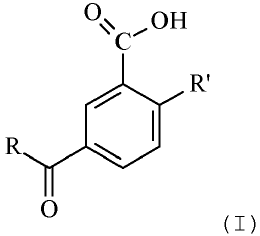 dans laquelle :
- le radical R désigne une chaîne aliphatique ayant de 2 à 22 atomes de carbone, saturée, linéaire, ramifiée ou cyclique ; une chaîne insaturée ayant de 2 à 22 atomes de carbone contenant une ou plusieurs doubles liaisons pouvant être conjuguées ; un noyau aromatique lié au radical carbonyle directement ou par l'intermédiaire de chaînes aliphatiques saturées ou insaturées ayant de 2 à 7 atomes de carbone ; lesdits noyaux et chaînes pouvant être substitués par un ou plusieurs substituants, identiques ou différents, choisis parmi (a) les atomes d'halogène, (b) le groupe trifluorométhyle, (c) des groupes hydroxyle sous forme libre ou estérifiée par un acide ayant de 1 à 6 atomes de carbone, ou (d) une fonction carboxyle sous forme libre ou estérifiée par un alcool inférieur ayant de 1 à 6 atomes de carbone ;
- R' est un groupe hydroxyle ou un groupe ester de formule 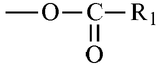 dans laquelle R₁ désigne une chaîne aliphatique, saturée ou insaturée, linéaire ou ramifiée contenant de 1 à 18 atomes de carbone ;
- ainsi que ses sels issus d'une base minérale ou organique.
